Europäisches Patentamt

(19) European Patent Office

Office européen des brevets

(11) Publication number: **0 098 039**
A2

## EUROPEAN PATENT APPLICATION

(12)

(21) Application number: 83302600.8

(22) Date of filing: 09.05.83

(51) Int. Cl.³: **B 01 J 27/18**
C 07 C 51/25, C 07 C 51/215
C 07 C 57/145

(30) Priority: 30.06.82 US 394031

(43) Date of publication of application:
11.01.84 Bulletin 84/2

(84) Designated Contracting States:
BE DE FR GB IT NL

(71) Applicant: The Standard Oil Company
Patent & License Division Midland Building
Cleveland Ohio 44115(US)

(72) Inventor: Dria, Dennis Edward
3604 Ambleside
Austin, Texas 78759(US)

(72) Inventor: Bremer, Noel Jerome
3633 By Lane
Kent Ohio 44240(US)

(74) Representative: Smith, Sydney et al,
Elkington and Fife High Holborn House 52/54 High
Holborn
London WC1V 6SH(GB)

(54) Preparation of improved mixed vanadium phosphorus oxide catalysts and their use in oxidation processes.

(57) Vanadium phosphorus mixed oxide containing catalysts are prepared in an organic liquid reaction medium capable of reducing the vanadium to a valence state of approximately +4 to form a non-solubilized catalyst precursor, contacting the non-solubilized catalyst precursor containing organic liquid with water to form a two phase system having an upper organic liquid phase and a lower non-solubilized catalyst precursor containing aqueous phase, drying the catalyst precursor and calcining. The vanadium phosphorus oxide catalysts so obtained are useful in the production of maleic anhydride from 4 carbon atom hydrocarbons.

EP 0 098 039 A2

Title: PREPARATION OF IMPROVED MIXED VANADIUM
PHOSPHORUS OXIDE CATALYSTS AND THEIR USE
IN OXIDATION PROCESSES

This invention relates to a method for preparing catalysts useful in the production of dicarboxylic acid anhydrides by the oxidation of hydrocarbons. More particularly it is directed to the preparation of catalysts suitable for producing maleic anhydride from 4-carbon atom hydrocarbons, such as n-butane, n-butenes, 1,3-butadiene or a mixture thereof.

Catalysts containing vanadium and phosphorus oxides have been used in the oxidation of 4-carbon atom hydrocarbons, such as n-butane, n-butenes, 1,3-butadiene or mixtures thereof with molecular oxygen or oxygen-containing gas to produce maleic anhydride. Conventional methods of preparing these catalysts involve reducing a pentavalent vanadium compound, and combining the same with a phosphorus compound, and if desired, promoter element compounds, under conditions which will provide or maintain vanadium in a valence state below +5 to form catalysts precursors capable of being converted to an oxide. The catalysts oxide precursor is then recovered and calcined to provide active catalytic material.

The use of gaseous HCl as a reducing agent for vanadium is disclosed in U.S. Patent No. 4,002,650 where the vanadium and phosphorus components are reacted in an aqueous solution. The use of gaseous HCl as a

reducing agent for vanadium is also described in U.S. Patent No. 4,043,943 where the vanadium and phosphorus components are reacted in liquid organic medium.

U.S. Patent No. 4,016,105 describes the preparation of vanadium and phosphorus oxide-containing catalysts, utilizing as reducing agents, organic acids or aldehydes, together with a co-reducing secondary alcohol. These reducing agents are added to an aqueous solution with the vanadium and phosphorus components.

Similar preparational techniques are described in European Patent Application No. 3,431 in which the additional step of comminuting the vanadium-phosphorus precursor to a particle size of 500 to 700 microns (0.5 to 0.7 mm) is disclosed.

The use of such reducing agents as disclosed in the art, requires special precautions in the preparation of these catalysts because of the corrosive nature of the materials utilized. The process of the present invention permits the preparation of mixed vanadium phosphorus oxide catalyst without the use of corrosive reducing agents.

A method for preparing catalysts containing vadadium and phosphorus oxides was described in U.S. Patent No. 4,132,670 which required the maintenance of a solid phase and dispersion of the vanadium-containing feed compound. The method includes forming a vanadium-containing compound dispersion in an organic liquid medium such as alcohols, aldehydes, ketones, ethers or mixtures thereof, heating the dispersion to reduce the vanadium, and thereafter adding phosphoric acid in an organic solvent.

The preparation of oxidation catalysts containing the mixed oxides of vanadium and phosphorus is disclosed in U.S. Patent No. 4,244,879 wherein a vanadium compound is at least partially solubilized in an organic liquid medium capable of reducing at least a portion of the vanadium to a +4 valence state, and unsolubilized vanadium having a particle size larger than about 0.1 mm diameter is removed from the medium before addition of a phosphorus-containing compound.

The preparation of vanadium phosphorus mixed oxide containing catalysts is disclosed in U.S. Patent No. 4,333,853, wherein partial reduction of a pentavalent vanadium compound is effected in the presence of a phosphorus compound in an organic liquid medium capable of reducing the vanadium.

U.S. Patent No. 4,317,778 discloses the preparation of vanadium phosphorus oxide catalysts utilizing a mixed phosphorus component compound source, preparing the catalyst in a liquid medium capable of reducing the vanadium component.

U.S. Patent No. 4,360,453 discloses the preparation of vanadium phosphorus oxide catalysts in which the precursor is solubilized by corrosive reducing agents in an organic liquid, and is recovered by contacting the solubilized precursor containing organic liquid with water, and thereafter separating the solubilized precursor containing aqueous phase from the organic phase, and drying.

## DISCLOSURE OF THE INVENTION

The process of the present invention includes the preparation of vanadium and phosphours mixed oxide containing catalysts in which a non-solubilized vanadium phosphorus oxide catalyst precursor is formed in an organic liquid reaction medium. Previously, the non-solubilized precursor was recovered from the reaction medium by methods including the evaporation or distillation of the organic liquid, or by filtering the non-solubilized precursor from the reaction medium to obtain a wet filter cake, and thereafter drying the catalyst precursor or wet filter cake to drive off the remaining organic liquid. In drying materials which contain appreciable amounts of organic liquids, special precautions must be taken with respect to the organic vapors evolved.

In the process of the present invention, the evolution of high concentrations of organic vapor is avoided. The non-solubilized catalyst precursor containing organic liquid reaction medium is contacted with a volume of water sufficient to form a two-phase system comprising a top, organic layer containing a substantial portion of said organic liquid and a bottom, non-solubilized catalyst precursor containing aqueous layer. The catalyst which is obtained by utilizing the process of the present invention is more highly active for oxidation reactions than vanadium and phosphorus oxide catalyst which are recovered from solubilized catalyst precursors which have been formed in an organic liquid medium and later contacted with water to separate a solubilized catalyst precursor containing aqueous phase from the organic liquid.

Surprisingly, the catalysts which are formed according to the process of the present invention exhibit activity which is equal to or greater than that activity exhibited by vanadium phosphorus oxide catalysts recovered from non-solubilized catalyst precursors prepared in organic liquid media but which have been recovered by the distillation, filtering and/or evaporation of the organic liquid. Although the presence of water in the reaction media during the preparation of vanadium phosphorus mixed oxide catalysts is detrimental to activity, we have found that after the non-solubilized vanadium phosphorus oxide precursor has been formed in the organic liquid medium in the absence of substantial amounts of water, the further processing according to the process of the present invention to recover the catalyst precursor is not detrimental to the activity of the final catalyst.

It is therefore an object of the present invention to provide a process for preparing vanadium and phosphorus mixed oxide containing catalysts useful for the oxidation of 4 carbon atom hydrocarbons to produce maleic anhydride, which catalyst exhibit excellent yields and selectivity to maleic anhydride.

It is a further object of the present invention to provide the process of preparing vanadium and phosphorus mixed oxide containing catalysts useful for the oxidation of 4 carbon atom hydrocarbons to produce maleic anhydride which is simplified, highly reproducible, and economical; which avoids the hazards of corrosion, and which is capable of commercial scale up.

## SUMMARY OF THE INVENTION

In general, the process of the present invention includes:

a)      introducing a pentavalent vanadium compound into an organic liquid reaction medium capable of reducing at least a portion of the vanadium to a valence state of about +4, in the absence of corrosive reducing agents;

b)      introducing at least one pentavalent phosphorus compound into the reaction medium;

c)      effecting reduction of the vanadium prior or subsequent to the addition of the phosphorus compound and reacting the vanadium with the phosphorus compound to form a non-solubilized catalyst precursor;

d)    contacting the non-solubilized catalyst precursor containing organic liquid medium with a volume of water sufficient to form a two-phase system comprising a top, organic phase containing a substantial portion of said organic liquid and a bottom, non-solubilized catalyst precursor containing aqueous phase;

e)    drying the catalyst precursor;

f)    calcining the catalyst precursor.

The invention therefore provides a process for the preparation of vanadium phosphorus mixed oxide containing catalysts wherein a non-solubilized vanadium phosphorus oxide catalyst precursor  is formed in an organic liquid reaction medium, in which the non-solubilized catalyst precurosr containing organic liquid reaction medium is contacted with a volume of water sufficient to form a two-phase system comprising a substantial portion of said organic liquid in an upper organic layer, and a lower catalyst precursor containing aqueous layer, and said lower layer is separated from said upper layer to recover the catalyst precursor.

The catalyst prepared by the process of the invention are particularly effective in the oxidation of 4-carbon atom hydrocarbons such as n-butane, n-butenes, 1,3-butadiene or mixtures thereof with molecular oxygen or an oxygen-containing gas in the vapor phase to produce high yields of maleic anhydride with high selectivity. Essentially all the product  produced in this oxidation process is maleic anhydride, with only minor amounts of lower acids being detected.

- 7 -                                    0098039

The present invention further provides vanadium and phosphorus mixed oxide containing catalysts prepared by the above process.

## DETAILED DESCRIPTION OF THE INVENTION

In the process for preparation of an oxidation catalyst containing the mixed oxides of vanadium and phosphorus, a vanadium compound, particularly a pentavalent vanadium compound, is introduced into a liquid reaction medium capable of reducing the valence state of the vanadium. Suitable vanadium compounds containing pentavalent vanadium include: vanadium pentoxide or vanadium salts, such as ammonium metavanadate. Vanadium pentoxide is preferred.

According to a preferred method of the present invention, at least one phosphorus-containing compound is added to the reaction medium before substantial reduction of the vanadium to a valence state less than +5 is effected. The phosphorus compounds utilized in the process of the invention are preferably pentavalent and suitable phosphorus compounds containing pentavalent phosphorus include: phosphoric acid, phosphorus pentoxide or a mixed pentavalent phosphorus component comprising a mixture of orthophosphoric acid and pyrophosphoric acid. Optionally, minor amounts of higher polyphosphoric acids may be included. The phosphorus component mixture should comprise 45 to 90 percent orthophosphoric acid, 10 to 50 percent pyrophosphoric acid, and 0 to 10 percent triphosphoric acid and higher polyphosphoric acids, percentages being based upon weight of total phosphoric acids. As hydrolysis is a factor in determining the ratio of orthophosphoric acid to pyrophosphoric acid when present in aqeuous solution, the above weight ratios are significant provided an extended period of hydrolysis has not occurred to convert the pyrophosphoric acid and higher polyphosphoric acids to the orthophosphoric form.

The phosphorus-containing compound or compounds may be added to the vanadium/liquid reaction medium in the form of a solution of the phosphorus component in either a component of the liquid reaction medium, or in a liquid capable of yielding the phosphorus component to the liquid reaction mixture. Alternatively, a vanadium compound and a phosphorus compound, such as 100% ortho-phosphoric acid, may be introduced simultaneously into the liquid reaction medium. In yet another mode, the vanadium compound is introduced into a solution or dispersion of the phosphorus component in the liquid reaction medium.

It is preferred that the vanadium-containing compound which is introduced into the liquid medium have a small particle size, and methods for further reducing particle size of the vanadium compound while in the liquid medium, such as by ball milling the initial suspension of vanadium in the liquid medium, may be employed.

The organic liquid medium employed in the process of the present invention must be capable of reducing at least a portion of the vanadium to a +4 valence state, preferably upon mixing and heating. In addition, the liquid medium should be a solvent for phosphoric acid and be relatively unreactive towards phosphoric acid. The liquid medium should not, however, be a solvent for the mixed oxide precursor of vanadium and phosphorus and thus the medium is maintained free of corrosive reducing or solubilizing agents such as HCl, HBr and oxalic acid.

Suitable organic liquid media for use in the invention include alcohols, aldehydes, ethers, glycols, ketones, halogenated olefins, mixtures thereof, and are preferably anhydrous. Suitable alcohols may be primary or secondary, saturated or unsaturated. Examples of organic liquids suitable for use in this invention include but are not limited to isopropanol, isobutanol, sec-butanol, allyl alcohol, crotyl alcohol, acetaldehyde, methyl ethyl ketone, ethylene glycol, dibutyl ether, hexachlorobutadiene and perchloropropene.

After the pentavalent vanadium compound is introduced into the liquid reaction medium, reduction of the vanadium is effected either prior to or subsequent to the addition of the phosphorus component to the liquid reaction medium. The reduction is preferably effected by heating the reaction medium, with stirring if desired.

According to the process of the present invention, the vanadium phosphorus catalyst precursor is recovered by contacting the non-solubilized catalyst precursor containing organic liquid reaction medium with a volume of water sufficient to form a two-phase system comprising a substantial portion of the organic liquid in a top, organic layer and a bottom, catalyst precursor containing aqueous layer. The upper layer contains substantially the organic liquid, possibly admixed with a small amount of water. The lower aqueous layer may include a small quantity of organic liquid, either due to partial miscibility of the liquids or to the organic liquid being trapped within the pores of the catalyst precursor. The organic liquid can be separated from the two phase system by conventional means such as decantation. Alternatively, after contacting the non-solubilized catalyst precursor containing organic liquid with water, the organic liquid and water/organic liquid azeotrope can be distilled away until substantially all the organic liquid has been removed.

In yet another alternative mode of the invention, the volume of the non-solubilized catalyst precursor containing organic liquid may be reduced, such as by centrifugation, evaporation or distillation to form a slurry, which is thereafter contacted with water for the separation. Preferably, water is added to the organic slurry in consecutive steps with intermediate decantations to remove the organic liquid as a water/. organic layer. One method of reducing the volume of the organic liquid to be separated includes the filtration of the non-solubilized vanadium phosphorus catalyst precursor from the organic liquid to obtain a wet filter cake. The filter cake may be optionally washed with fresh organic liquid to remove organic redox products from the vanadium reduction step, prior to contacting with water to recover the non-solubilized catalyst precursor.

After the non-solubilized catalyst precursor is recovered into the aqueous phase, excess water may be removed by conventional methods such as filtration, and/or the aqueous/non-solubilized catalyst precursor mix may be subected to drying techniques. These include oven drying, spray drying, and the like. After the catalyst precursor is dried, it is then calcined.

Preferred vanadium and phosphorus oxide catalysts for the oxidation of 4-carbon atom hydrocarbons to maleic anhydride contain vanadium in an average valence state of +3.5 to +4.6. This average valence state is achieved when at least a portion of the pentavalent vanadium introduced into the reaction mixture is reduced to the +4 state. The average valence state of the vanadium is reduced preferably to approximately +4.1. After partial reduction of the vanadium, in one embodiment of the invention where reduction is effected prior to the addition of the phosphorus component to the reaction medium, large particle unsolubilized vanadium-containing compounds may be removed from the reaction mixture as taught in U.S. Patent No. 4,244,879. In one embodiment of the invention, during the reduction of the vanadium in the organic liquid medium with heating under reflux conditions, organic liquid is removed by distillation during reaction of the vanadium.

It is within the scope of this invention to include promoter element-containing compounds in the catalyst at a suitable point, such as either by introducing the promoter element containing compounds in the organic liquid medium prior or subsequent to the reduction of the vanadium, in order that the partially reduced catalyst precursor contain the promoter element. Additionally, promoter elements may be added to the catalyst precursor after drying or to the catalyst after calcination. Suitable promoters include but are not limited to alkali metals, alkaline earth metals, Ti, Cr, W, Nb, Ta, Mn, Th, U, Co, Mo, Fe, Zn, Hf, Zr, Ni, Cu, As, Sb, Te, Bi, Sn, Ge, Cd, lanthanides or mixtures thereof. Suitable promoter element containing compounds include metal oxides, hydroxides, and salts such as nitrates, carbonates and acetates.

Catalysts produced by the process of this invention are prepared preferably to exhibit a phosphorus to vanadium ratio of 0.8:1 to 1.3:1. The molar ratio of promoter to vanadium is generally between 0.01 to 0.5. The catalyst is generally calcined in an inert atmosphere, air or an oxygen-containing gas at a

temperature of 250$^{\circ}$C to 600$^{\circ}$C. Calcination of the catalyst may also be accomplished by heating the catalyst in a mixture of steam and air or air alone at a temperature of 300$^{\circ}$C to 500$^{\circ}$C. The catalyst may also be calcined either in the presence of hydrocarbon, an inert gas, or both.

The hydrocarbon reacted to form maleic anhydride may be n-butane, the n-butenes, 1,3-butadiene or a mixture thereof. The use of n-butane or a mixture of hydrocarbons that are produced in refinery streams is preferred. The molecular oxygen needed for the reaction to produce maleic anhydride is most conveniently added as air, but synthetic streams containing molecular oxygen are also suitable. In addition to the hydrocarbon and molecular oxygen, other gases may be added to the reactant feed. For example, steam or nitrogen could be added to the reactants.

The ratio of the reactants may vary widely and are not critical. The ratio of molecular oxygen to the hydrocarbon may range from 3 to 30 moles of oxygen per mole of hydrocarbon. Preferred oxygen/ hydrocarbon ratios are 4 to 20 moles of oxygen per mole of hydrocarbon.

The reaction temperature may vary widely and is dependent upon the particular hydrocarbon and catalyst employed. Normally, temperatures of 250$^{\circ}$C to 600$^{\circ}$C are employed with temperatures of 325$^{\circ}$C to 475$^{\circ}$C being preferred.

The catalyst may be used alone or a support could be employed. Suitable supports include silica, alumina, Alundum, alumina-silica, silicon carbide, titania, boron phosphate and zirconia. The catalyst may be used in a fixed-bed reactor using tablets, pellets or the like, or in a fluid-bed reactor using catalysts prepared such as by spray drying or the oil drop technique, and preferably having a particle size of less than 300 microns. The contact time may be as low as a fraction of a second or as high as 50 seconds. The reaction may be conducted at atmospheric, superatmospheric or subatmospheric pressure.

SPECIFIC EMBODIMENTS OF THE INVENTION

Example 1

A catalyst of the formual $V_1P_{1.2}O_x$ (where x is the number of oxygens required to satisfy the valence requirements of the other elements) was prepared by the following method.

The catalyst precursor was prepared by introducing 7.3 parts of $V_2O_5$ and 10.5 parts mixed phosphoric acid into 100 parts isobutanol with stirring, and refluxing the resulting slurry for 6 hours. The mixed phosphoric acid source contained 87% ortho-phosphoric acid, 12% pyro-phosphoric acid and 1% higher phosphoric acids based upon total weight of phosphoric acid. A slurry of non-solubilized vanadium phosphoric catalyst precursor in isobutanol resulted, which was allowed to cool.

The non-solubilized, vanadium phosphorus catalyst precursor containing organic liquid was filtered to remove excess isobutanol, forming a wet filter cake containing 75% solids. 14 parts of wet filter cake was contacted with 11.2 parts of deionized water. The resulting isobutanol rich top layer was decanted off of the two phase system which was formed. A non-solubilized vanadium phosphorus catalyst precursor remained in the aqueous bottom layer. An additional 6.6 parts of deionized water was added to the remaining mixture, and the mixture was spray dried to form microspheroidal catalyst particles. The catalyst was

- 14 -                          0098039

then calcined at 415$^O$C for 2 hours.

<u>Comparative Example 2</u>

A $V_1P_{1.2}O_x$ catalyst was prepared according to the procedure of example 1, except that after filtration the isobutanol remaining in the wet filter cake was removed by evaporative drying. The thoroughly dried catalyst precursor was introduced into water with mixing, and the resulting aqueous slurry was spray dried to form microspheroidal catalyst particles.

The catalyst of examples 1 and 2 were tested for the oxidation of n-butane to produce maleic anhydride using a 1-1/2 inch diameter fluid-bed reactor. The reactor consisted of a 61cm length of stainless steel tubing having an outer diameter of 3.8cm, having a stainless steel sparger at the bottom of the tube to act as a gas distributor, with an axial 0.64cm outer diameter thermo well and a separate hydrocarbon inlet at the bottom of the tube. The reactor was fitted with internal gas redistributing baffles. Gas preheating and reactor temperature was accomplished by placement of the reactor unit in a thermostated fluidized sand bath. Flasks for receiving the product maleic anhydride were air cooled and tail gases were routed to a gas chromatograph for analysis. Reaction conditions and results of the tests run are described in the Table. The results are stated in terms as follows.

$$\text{Single Pass Yield} = \frac{\text{Moles of Maleic Anhydride Formed}}{\text{Moles of Butane Fed}} \times 100$$

$$\text{Total Conversion} = \frac{\text{Moles of Butane Reacted}}{\text{Moles of Butane Fed}} \times 100$$

$$\text{Selectivity} = \frac{\text{Single Pass Yield}}{\text{Total Conversion}}$$

Contrary to earlier belief that introduction of the vanadium phosphorus oxide catalyst precursor into water prior to the complete drying of the catalyst precursor to remove organics would lead to a substantial loss in activity of the resulting vanadium phosphorus oxide containing catalyst, it has been found that after the formation of the vanadium phosphorus catalyst precursor, water may be used to separate the non-solubilized vanadium phosphorus catalyst precursor from the organic liquid medium by introduction of water into the precursor/ organic liquid mixture to form a binary phase system. This procedure, as demonstrated in the Table, surprisingly results in no decrease in the activity of the catalyst thus formed, as shown by a comparison of the results for example 1 and 2.

Examples 3-8

Catalysts of the formula $V_1P_{1.2}O_x$ were prepared according to the procedure of example 1. These catalyst were tested for the oxidation of n-butane to produce maleic anhydride according to the test procedures for examples 1 and 2. Results of the tests and test conditions are contained in the Table.

## TABLE

### PRODUCTION OF MALEIC ANHYDRIDE FROM N-BUTANE USING $V_1P_{1.2}O_x$ CATALYSTS.

| Example No. | Bath/Bed Temperature (°C) | Air/Hydrocarbon Ratio | % Conversion | Maleic Anhydride | |
|---|---|---|---|---|---|
| | | | | % Yield | % Selectivity |
| 1 | 420/433 | 30/1 | 88 | 55.4 | 63 |
| C2 | 420/432 | 30/1 | 85.4 | 54.7 | 64.1 |
| 3 | 390/395 | 31/1 | 78.3 | 54.5 | 69.6 |
| 4 | 401/407 | 30/1 | 80.2 | 53.2 | 66.3 |
| 5 | 414/424 | 27/1 | 86.8 | 54.3 | 62.6 |
| 6 | 420/431 | 32/1 | 91.4 | 55.1 | 60.3 |
| 7 | 421/435 | 28/1 | 83.9 | 52.1 | 62.1 |
| 8 | 421/435 | 29/1 | 86.5 | 53.2 | 61.5 |

CLAIMS:

1. A process for the preparation of vanadium phosphorus mixed oxide containing catalysts wherein a non-solubilized vanadium phosphorus oxide catalyst precursor is formed in an organic liquid reaction medium, in which the non-solubilized catalyst precursor containing organic liquid reaction medium is contacted with a volume of water sufficient to form a two-phase system comprising a substantial portion of said organic liquid in an upper organic layer, and a lower catalyst precursor containing aqueous layer, and said lower layer is separated from said upper layer to recover the catalyst precursor.

2. A process for the preparation of vanadium phosphorus mixed oxide containing catalysts comprising:

   a) introducing a pentavalent vanadium compound into an organic liquid reaction medium capable of reducing at least a portion of the vanadium to a valence state of approximately +4, in the absence of corrosive reducing agents;

   b) introducing at least one pentavalent phosphorus compound into the reaction medium;

   c) effecting reduction of the vanadium prior or subsequent to the addition of the phosphorus compound and reacting the vanadium with the phosphorus compound to form a non-solubilized catalyst precursor, in which wherein the non-solubilized catalyst precursor containing organic liquid reaction medium is contacted with a volume of water sufficient to form a two-phase system comprising an upper organic layer containing a substantial portion of said organic liquid and a bottom catalyst precursor containing aqueous layer.

- 18 -                              0098039

3.   A process for the preparation of a vanadium
phosphorus mixed oxide containing oxidation catalyst
which comprises:

   a)   introducing a pentavalent vanadium compound
into an organic liquid reaction medium capable of
reducing at least a portion of the vanadium to a
valence state of approximately +4, in the absence of
corrosive reducing agents;

   b) introducing at least one pentavalent phosphorus
compound into the reaction medium;

   c) effecting reduction of the vanadium prior or
subsequent to the addition of the phosphorus compound
and reacting the vanadium with the phosphorus
compound to form a non-solubilized catalyst precursor;

   d)   contacting the non-solubilized catalyst
precursor containing organic liquid reaction medium
with a volume of water sufficient to form a two phase
system comprising an upper organic layer containing a
substantial portion of said organic liquid and a lower
non-solubilized catalyst precursor containing aqueous
layer;

   e) drying the catalyst precursor and

   f) calcining the catalyst precursor.


4.   A process as claimed in claim 2 or claim 3
characterised in that the volume of the organic
liquid reaction medium is reduced prior to contacting
the non-solubilized catalyst precursor containing
organic liquid reaction medium with water.


5.   A process as claimed in claim 4 characterised in
that the volume of the organic liquid reaction
medium is reduced by distillation or evaporation.

6. A process as in claim 4 characterised in that
the volume of the organic liquid reaction medium is
reduced by filtering the non-solubilized catalyst
precursor containing organic liquid reaction medium
to obtain a filter cake wet with said organic
liquid and thereafter contacting said wet filter
cake with water.

7. A process as in claim 6 characterised in that
said wet filter cake is washed with a portion of said
organic liquid prior to contacting said wet filter
cake with water.

8. A process as in claim 2 or claim 3 characterised in
that a portion of said organic liquid and water is
removed from the two phase system by distillation.

9. A process as claimed in claim 2 or claim 3
characterised in that said reduction of vanadium is
effected by heating at reflux conditions.

10. A process as claimed in claim 2 or claim 3
characterised in that the phosphorus compound is added
to the reaction medium before substantial reduction
of vanadium occurs.

11. A process as claimed in claim 2 or claim 3
characterised in that the organic liquid is selected
from isopropanol, isobutanol, sec-butanol, allyl
alcohol, crotyl alcohol, acetaldehyde, methyl ethyl
ketone, ethylene glycol, dibutyl ether, hexachlorobutadiene, and perchloropropene.

12. A process as claimed in claim 2 or claim 3
characterised in that the catalyst additionally comprises promoter elements selected from alkali metals, alkaline earth metals, Ti, Cr, W, Nb, Ta, Mn, Th, U, Co, Mo, Fe, Zn, Hf, Zr, Ni, Cu, As, Sb, Te, Bi, Sn, Ge, Cd, lanthanides or mixtures thereof.

13.  A process for the production of maleic anhydride by the oxidation of 4 carbon atom hydrocarbons with molecular oxygen or an oxygen containing gas in the vapor phase at a reaction temperature of $250^{\circ}C$ to $600^{\circ}C$ in the presence of a catalyst containing the mixed oxides of vanadium and phosphorus characterised in that the catalyst has been prepared by a process as claimed in any of claims 1 to 12.